(19) Europäisches Patentamt
European Patent Office
Office européen des brevets

(11) **EP 2 471 392 B1**

(12) **EUROPEAN PATENT SPECIFICATION**

(45) Date of publication and mention
of the grant of the patent:
**11.09.2013 Bulletin 2013/37**

(51) Int Cl.:
***A24F 47/00*** *(2006.01)*

(21) Application number: **12161883.9**

(22) Date of filing: **04.03.2009**

(54) **An aerosol generating system having a controller for controlling the formation of smoke constituents**

Aerosolerzeugungssystem mit einem Kontroller zum Steuern der Bildung von Rauchbestandteilen

Système de génération d'aérosol comprenant un régulateur pour contrôler la formation de constituants de fumée

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **25.03.2008 EP 08251039**

(43) Date of publication of application:
**04.07.2012 Bulletin 2012/27**

(62) Document number(s) of the earlier application(s) in accordance with Art. 76 EPC:
**09725591.3 / 2 257 195**

(73) Proprietor: **Philip Morris Products S.A.**
**2000 Neuchâtel (CH)**

(72) Inventors:
• **Greim, Olivier**
**1423 Villars-Burquin (CH)**
• **Fernando, Felix**
**Wokingham, Berkshire RG40 2LU (GB)**
• **Radtke, Falk**
**D-53797 Lohmar (DE)**

(74) Representative: **Loustalan, Paul William**
**Reddie & Grose LLP**
**16 Theobalds Road**
**London WC1X 8PL (GB)**

(56) References cited:
EP-A- 0 438 862    WO-A-03/070031
US-A- 5 144 962    US-A- 6 040 560

**Description**

This invention relates to electrically heated aerosol generating systems and in particular to the control of smoke constituents released by an electrically heated aerosol generating system on heating.

Traditional cigarettes deliver smoke as a result of combustion of the tobacco and the wrapper which occurs at temperatures which may exceed 800 degrees Celsius during a puff. At these temperatures, the tobacco is thermally degraded by pyrolysis and combustion. The heat of combustion releases and generates various gaseous combustion products and distillates from the tobacco. The products are drawn through the cigarette and cool and condense to form a smoke containing the tastes and aromas associated with smoking. At combustion temperatures, not only tastes and aromas are generated but also a number of undesirable compounds.

Electrically heated smoking systems are known, which operate at lower temperatures. An example of such an electrical smoking system is disclosed in the International patent application WO03/070031. This electrical smoking system uses a controller to control the amount of electric power delivered to the heating elements in response to the battery discharge cycle.

Furthermore, commonly assigned U.S. Patent Nos. US-A-5,060,671; US-A-5,144,962; US-A-5,372,148; US-A-5,388,594; US-A-5,498,855; US-A-5,499,636; US-A-5,505,214; US-A-5,530,225; US-A-5,591,368; US-A-5,665,262; US-A-5,666,976; US-A-5,666,978; US-A-5,692,291; US-A-5,692,525; US-A-5,708,258; US-A-5,750,964; US-A-5,902,501; US-A-5,915,387; US-A-5,934,289; US-A-5,954,979; US-A-5,967,148; US-A-5,988,176; US-A-6,026,820 and US-A-6,040,560 disclose electrical smoking systems and methods of manufacturing such an electrically smoking system.

A method of controlling the release of volatile compounds from an electrically heated aerosol generating system is described herein. The electrically heated aerosol generating system comprises a supply of electrical energy, at least one heating element connected to the supply of electrical energy and an aerosol-forming substrate. The aerosol-forming substrate releases a plurality of volatile compounds upon heating, wherein each of the plurality of volatile compounds has a minimum release temperature above which the volatile compound is released. The method comprises the step of selecting a predetermined maximum operation temperature. This predetermined maximum operation temperature is below the minimum release temperature of at least one of the plurality of volatile compounds in order to prevent its release from the aerosol-forming substrate. The method further comprises the step of controlling the temperature of the at least one heating element such that at least one of the volatile compounds is released. This step of controlling comprises measuring of the resistivity of the at least one heating element and deriving a value of actual operation temperature of the at least one heating element from the measurement of resistivity. Furthermore, the step of controlling comprises comparing the value of the actual operation temperature of the at least one heating element with the predetermined maximum operation temperature. Furthermore, the step of controlling comprises adjusting the electrical energy supplied to the at least one heating element in order to keep the actual operation temperature of the at least one heating element at or below the predetermined maximum operation temperature.

Preferably, the step of controlling comprises adjusting the electrical energy supplied to the at least one heating element in order to keep the actual operation temperature of the at least one heating element in a temperature range below the predetermined maximum operation temperature. The predetermined temperature range may be up to the predetermined maximum operation temperature.

Preferably, the step of controlling is repeated as often as necessary during heating of the at least one heating element.

Embodiments of the invention have the advantage that the actual operation temperature of the at least one heating element can be controlled such that pyrolysis or combustion of the aerosol-forming substrate is prevented. This allows to reduce the number of volatile components released or formed during the heating. As the formation of harmful substances typically takes place at elevated temperatures during pyrolysis and combustion, the formation of theses harmful components, for example formaldehyde, is significantly reduced by the method described herein.

Moreover, the need for thermistors or other sensors that take up some of the limited space within an electrically heated aerosol generating system is avoided. This makes embodiments of the invention particularly suitable for use in electrically heated aerosol generating systems of the type having several heating elements, in which the temperature of each heating element is controlled. Embodiments of the invention have the further advantage that calculation of temperature from resistivity may be performed within existing controllers. This makes these embodiments of the invention straightforward and cost effective to implement. Additionally, the invention avoids complications in the attachment of a temperature sensor that provides good and reliable thermal contact with the at least one heating element.

The aerosol-forming substrate preferably comprises a tobacco-containing material containing volatile compounds which are released from the substrate upon heating. Alternatively, the aerosol-forming substrate may comprise a non-tobacco material such as those used in the devices of EP-A-1 750 788 and EP-A-1 439 876.

Preferably, the aerosol-forming substrate further comprises an aerosol former. Examples of suitable aerosol formers are glycerine and propylene glycol. Additional examples of potentially suitable aerosol formers are described in EP-A-0 277 519 and US-A-5,396,911.

The aerosol-forming substrate may be a solid substrate. The solid substrate may comprise, for example, one or more of: powder, granules, pellets, shreds, spaghettis, strips or sheets containing one or more of: herb leaf, tobacco leaf, fragments of tobacco ribs, reconstituted tobacco, homogenised tobacco, extruded tobacco and expanded tobacco. The solid substrate may be in loose form, or may be provided in a suitable container or cartridge. Optionally, the solid substrate may contain additional tobacco or non-tobacco volatile flavour compounds, to be released upon heating of the substrate.

Optionally, the solid substrate may be provided on or embedded in a thermally stable carrier. The carrier may take the form of powder, granules, pellets, shreds, spaghettis, strips or sheets. Alternatively, the carrier may be a tubular carrier having a thin layer of the solid substrate deposited on its inner surface, such as those disclosed in US-A-5,505,214, US-A-5,591,368 and US-A-5,388,594, or on its outer surface, or on both its inner and outer surfaces. Such a tubular carrier may be formed of, for example, a paper, or paper like material, a non-woven carbon fibre mat, a low mass open mesh metallic screen, or a perforated metallic foil or any other thermally stable polymer matrix.

The solid substrate may be deposited on the surface of the carrier in the form of, for example, a sheet, foam, gel or slurry. The solid substrate may be deposited on the entire surface of the carrier, or alternatively, may be deposited in a pattern in order to provide a nonuniform flavour delivery during use.

Alternatively, the carrier may be a non-woven fabric or fibre bundle into which tobacco components have been incorporated, such as that described in EP-A-0 857 431. The non-woven fabric or fibre bundle may comprise, for example, carbon fibres, natural cellulose fibres, or cellulose derivative fibres.

Alternatively, the carrier may be at least a part of the heating element of the electrically heated aerosol generating system. In such cases, the heating element is typically disposable. For example, the solid substrate may be deposited as a thin layer on a metallic foil or on an electrically resistive support as described in US-A-5 060 671.

The aerosol-forming substrate may alternatively be a liquid substrate. If a liquid substrate is provided, the electrically heated aerosol generating system preferably comprises means for retaining the liquid. For example, the liquid substrate may be retained in a container, such as that described in EP-A-0 893 071. Alternatively or in addition, the liquid substrate may be absorbed into a porous carrier material, as described in WO-A-2007/024130, WO-A-2007/066374, EP-A-1 736 062, WO-A-2007/131449 and WO-A-2007/131450. The porous carrier material may be made from any suitable absorbent plug or body, for example, a foamed metal or plastics material, polypropylene, terylene, nylon fibres or ceramic. The liquid substrate may be retained in the porous carrier material prior to use of the electrically heated aerosol generating system or alternatively, the liquid substrate material may be released into the porous carrier material during, or immediately prior to use. For example, the liquid substrate may be provided in a capsule, as described in WO-A-2007/077167. The shell of the capsule preferably melts upon heating and releases the liquid substrate into the porous carrier material. The capsule may optionally contain a solid in combination with the liquid.

If the aerosol-forming substrate is a liquid substrate, the electrically heated aerosol generating system may further comprise means for heating a small amount of liquid at a time. The means for heating a small amount of liquid at a time may include, for example, a liquid passageway in communication with the liquid substrate, as described in EP-A-0 893 071. The liquid substrate is typically forced into the liquid passageway by capillary force. The heating element is preferably arranged such that during use, only the small amount of liquid substrate within the liquid passageway, and not the liquid within the container, is heated and volatilised.

Alternatively, or in addition, if the aerosol-forming substrate is a liquid substrate, the electrically heated aerosol generating system may further comprise an atomiser in contact with the liquid substrate source and including the at least one heating element. In addition to the heating element, the atomiser may include one or more electromechanical elements such as piezoelectric elements. Additionally or alternatively, the atomiser may also include elements that use electrostatic, electromagnetic or pneumatic effects. The electrically heated aerosol generating system may still further comprise a condensation chamber.

The aerosol-forming substrate may alternatively be any other sort of substrate, for example, a gas substrate, or any combination of the various types of substrate. During operation, the substrate may be completely contained within the electrically heated aerosol generating system. In that case, a user may puff on a mouthpiece of the electrically heated aerosol generating system. Alternatively, during operation, the substrate may be partially contained within the electrically heated aerosol generating system. In that case, the substrate may form part of a separate article and the user may puff directly on the separate article.

The at least one heating element may comprise a single heating element. Alternatively, the at least one heating element may comprise more than one heating element. Preferably, the electrically heated aerosol generating system comprises two or more heating elements, for example two to twenty heating elements. The heating element or heating elements may be arranged appropriately so as to most effectively heat the aerosol-forming substrate.

The at least one heating element preferably comprises an electrically resistive material. Suitable electrically resistive materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics.

Examples of suitable doped ceramics include doped silicon carbides. Examples of suitable metals include titanium, zirconium, tantalum and metals from the platinum group. Examples of suitable metal alloys include stainless steel, nickel-, cobalt-, chromium-, aluminium-titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal® and iron-manganese-aluminium based alloys. In composite materials, the electrically resistive material may optionally be embedded in, encapsulated or coated with an insulating material or vice-versa, depending on the kinetics of energy transfer and the external physicochemical properties required. Examples of suitable composite heating elements are disclosed in US-A-5,498,855, WO-A-03/095688 and US-A-5,514,630.

The at least one heating element may take any suitable form. For example, the at least one heating element may take the form of a heating blade, such as those described in US-A-5,388,594, US-A-5,591,368 and US-A-5,505,214. Where the aerosol-forming substrate is a liquid provided within a container, the container may incorporate a disposable heating element. Alternatively, one or more heating needles or rods that run through the centre of the aerosol-forming substrate, as described in KR-A-100636287 and JP-A-2006320286, may also be suitable. Other alternatives include a heating wire or filament, for example a Ni-Cr, platinum, tungsten or alloy wire, such as those described in EP-A-1 736 065, or a heating plate. Optionally, the heating element may be deposited in or on a rigid carrier material.

The at least one heating element may heat the aerosol-forming substrate by means of conduction. The heating element may be at least partially in contact with the substrate, or the carrier on which the substrate is deposited. Alternatively, the heat from the heating element may be conducted to the substrate by means of a heat conductive element.

Alternatively, the at least one heating element may transfer heat to the incoming ambient air that is drawn through the electrically heated aerosol generating system during use, which in turn heats the aerosol-forming substrate by convection. The ambient air may be heated before passing through the aerosol-forming substrate, as described in WO-A-2007/066374. Alternatively, if the aerosol-forming substrate is a liquid substrate, the ambient air may be first drawn through the substrate and then heated, as described in WO-A-2007/078273.

In one preferred embodiment, the actual operation temperature is retrieved from a look-up table which stores resistivity and temperature relationships for the at least one heating element. In an alternative embodiment, the resistivity is determined by evaluating a polynomial of the form $p(T) = \rho_o{}^*(1 + \alpha_1 T + \alpha_2 T^2)$ where $p(T)$ is the measured resistivity of the at least one heating element or the plurality of heating elements, $\rho_o$ is a reference resistivity and $\alpha_1 + \alpha_2$ are polynomial coefficients. The evaluation may be performed by the controller. Alternatively, polynomial functions of higher degrees or other mathematical functions may be used to describe the variation of the resistivity of the at least one heating element as a function of temperature.

Alternatively, a piece-wise linear approximation may be used. This alternative simplifies and speeds up the calculation.

Preferably, the system comprises more than one heating element and the aerosol-forming substrate is arranged such that the aerosol-forming substrate is in thermal proximity with each of the heating elements.

The system is preferably provided with an aerosol-forming substrate detector and a puff detector so that electrical energy is only delivered to the at least one heating element when an aerosol-forming substrate is detected in place and a puff on the system is also detected.

Preferably, the at least one heating element comprises an iron aluminium alloy. This alloy shows a strong dependence between resistance and temperature that is useful to determine the temperature of the heating element by measuring its resistance. Alternatively, the heating element comprises the aforementioned electrical resistive materials other suitable materials that show a comparable thermal resistivity characteristic with a strong dependency of the resistivity on temperature.

Preferably, the step of controlling is performed with a frequency of about 100 Hz to about 10 kHz during a puff, preferably with a frequency of about 1 kHz.

The invention will be further described, by way of example only, with reference to the accompanying drawings in which:

Figure 1 is a schematic diagram of an electrically heated aerosol generating system with an aerosol-forming substrate inserted into the system;
Figure 2 is a graph showing changes in resistivity of the heater blades with temperature.

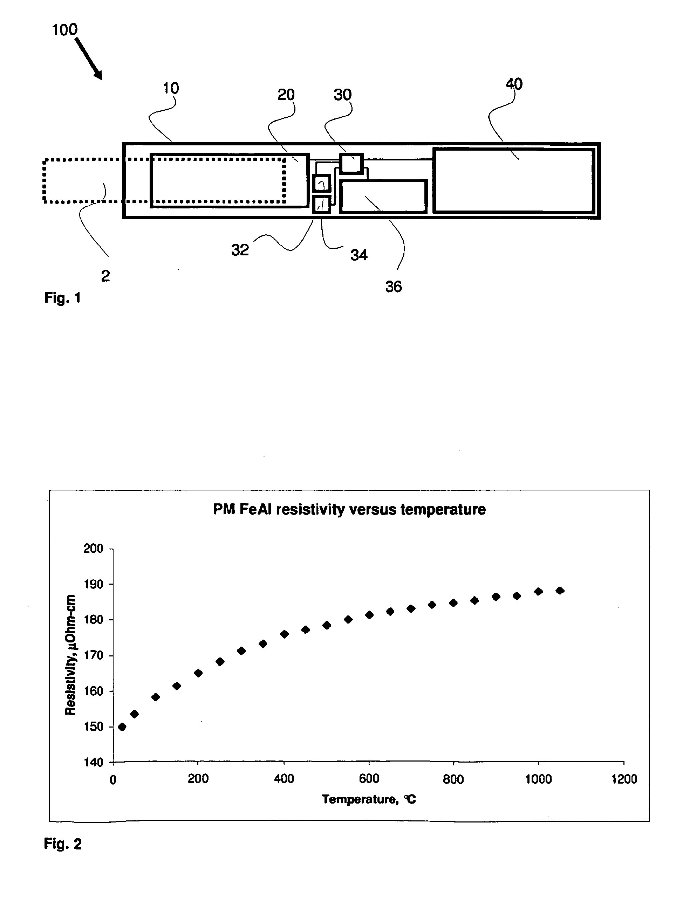

In Figure 1, the inside of an embodiment of the electrically heated aerosol generating system 100 is shown in a simplified manner. Particularly, the elements of the electrically heated aerosol generating system 100 are not drawn to scale. Elements that are not relevant for the understanding of the invention have been omitted to simplify Figure 1.

The electrically heated aerosol generating system 100 comprises a housing 10 and an aerosol-forming substrate 2, for example a cigarette. The aerosol-forming substrate 2 is pushed inside the housing 10 to come into thermal proximity with the heating element 20. The aerosol-forming substrate 2 will release a range of volatile compounds at different temperatures. Some of the volatile compounds released from the aerosol-forming substrate 2 are only formed through the heating process. Each volatile compound will be released above a characteristic release temperature. By controlling the maximum operation temperature of the electrically heated aerosol generating system 100 to be below the release temperature of some of the volatile compounds, the release or formation of these smoke constituents can be avoided.

Additionally, the housing 10 comprises an electrical energy supply 40, for example a rechargeable lithium ion battery. A controller 30 is connected to the heating element 20, the electrical energy supply 40, an aerosol-forming substrate detector 32, a puff detector 34 and a graphical user interface 36, for example a display.

The aerosol-forming substrate detector 32 detects the presence of an aerosol-forming substrate 2 in thermal proximity with the heating element 20 and signals the presence of an aerosol-forming substrate 2 to the controller 30.

The puff detector 34 detects airflow in the system, indicative of a puff being taken on the electrically heated aerosol generating system 100. The puff detector 34 signals such a puff to the controller 30.

The controller 30 controls the user interface 36 to display system information, for example, battery power, temperature, status of aerosol-forming substrate 2, other messages or combinations thereof.

The controller 30 further controls the maximum operation temperature of the heating element 20.

Figure 2 shows a graph of resistivity p (rho) against temperature for a typical iron aluminum (FeAl) alloy used as a heating element 20 in the embodiment described with reference to Figure 1. The actual resistivity p characteristic will vary depending on the exact composition of the alloy and the geometrical configuration of the heating element 20. The graph of Figure 2 is exemplary only.

Figure 2 shows that resistivity p increases with increasing temperature. Thus, knowledge of resistivity p at any given time can be used to deduce the actual operation temperature of the heating element 20.

The resistance of the heating element R = V/I; where V is the voltage across the heating element and I is the current passing through the heating element 20. The resistance R depends on the configuration of the heating element 20 as well as the temperature and is expressed by the following relationship:

$$R = \rho\,(T)\ \ast\ L/S \qquad\qquad \text{equation 1}$$

Where p (T) is the temperature dependent resistivity, L is length and S the cross-sectional area of the heating element 20. L and S are fixed for a given heating element 20 configuration and can be measured. Thus, for a given heating element design R is proportional to p (T).

The resistivity p(T) of the heating element can be expressed in polynomial form as follows:

$$\rho\,(T) = \rho_0 \ast (1 + \alpha_1\,T + \alpha_2\,T^2) \qquad\qquad \text{equation 2}$$

Where $\rho_0$ is the resistivity at a reference temperature To and $\alpha_1$ and $\alpha_2$ are the polynominal coefficients.

Thus, knowing the length and cross-section of the heating element 20, it is possible to determine the resistance R, and therefore the resistivity p at a given temperature by measuring the heating element voltage V and current I. The temperature can be obtained simply from a look-up table of the characteristic resistivity versus temperature relationship for the heating element being used or by evaluating the polynomial of equation (2) above. Preferably, the process may be simplified by representing the resistivity p versus temperature curve in one or more, preferably two, linear approximations in the temperature range applicable to tobacco. This simplifies evaluation of temperature which is desirable in a controller 30 having limited computational resources.

In a preparation of the controlling of the maximum operation temperature, a value for the maximum operation temperature of the electrically heated aerosol generating system 100 is selected. The selection is based on the release temperatures of the volatile compounds that should and should not be released. This predetermined value is then stored in the controller 30 along with an acceptable range, for example minus 5 percent of the predetermined maximum operation temperature.

The controller 30 heats the heating element 20 by supplying electrical energy to the heating element 20. Preferably, in order to save energy, the controller only heats the heating element 20, if the aerosol-forming substrate detector 32 has detected an aerosol-forming substrate 20 and the puff detector 34 has detected the presence of a puff.

In use, the controller 30 measures the resistivity p of the heating element 20. The controller 30 then converts the resistivity of the heating element 20 into a value for the actual operation temperature of the heating element, by comparing the measured resistivity p with the look-up table. In the next step, the controller 30 compares the derived actual operation temperature with the predetermined maximum operation temperature. If the actual operation temperature is below the lower range of the predetermined maximum operation temperature, the controller 30 supplies the heating element 20 with additional electrical energy in order to raise the actual operation temperature of the heating element 20. If the actual operation temperature is above the upper range of the predetermined maximum operation temperature, the controller 30 reduces the electrical energy supplied to the heating element 20 in order to lower the actual operation temperature back into the acceptable range of the predetermined maximum operation temperature.

The control of heating element temperature based on heating element resistivity p is not limited to the electrically heated aerosol generating system as described with respect to Figure 1 but is applicable to any electrically heated aerosol generating system in which a heating element 20 delivers heat energy to a tobacco or other aerosol-forming substrate to release volatile compounds.

Various others modifications are possible within the scope of the invention and will occur to those skilled in the art.

## Claims

1. An aerosol generating system (100) comprising:

at least one heating element (20) configured to receive an aerosol forming substrate (2); and
a controller (30) configured to control the release of volatile compounds from the aerosol generating system (100), wherein the aerosol-forming substrate (2) releases a plurality of volatile compounds upon heating, and wherein each of the plurality of volatile compounds has a minimum release temperature above which the volatile compound is released,
the controller (30) comprising:

means for selecting a predetermined maximum operation temperature, wherein the predetermined maximum operation temperature is below the minimum release temperature of at least one of the volatile compounds in order to prevent its release from the aerosol-forming substrate (2);
means for controlling the temperature of the at least one heating element (20) such that at least one volatile compound is released; and
means for measuring the resistivity of the at least one heating element (20),
wherein the controller (30) is further configured to:

derive a value of actual operation temperature of the at least one heating element (20) from the measurement of resistivity;
compare the value of actual operation temperature with the predetermined maximum operation temperature; and
adjust the temperature of the at least one heating element (20) in order to keep the actual operation temperature of the at least one heating element (20) below the predetermined maximum operation temperature.

2. A system according to claim 1, wherein the at least one heating element (20) comprises a ceramic material.

3. A system according to Claim 2, wherein the heating element (20) further comprises a metal.

4. A system according to claim 3, wherein the ceramic material and the metal form a composite material that comprises the at least one heating element (20).

5. A system according to claim 3 or 4, wherein the metal coats the ceramic material.

6. A system according to any of claims 3, 4 or 5, wherein the metal is from the platinum group.

7. A system according to any of claims 3 to 6, wherein the at least one heating element (20) is provided in the form of a heating blade.

8. A system according to any of the preceding claims, wherein the at least one heating element (20) is electrical.

9. A system according to Claim 8, further comprising a supply of electrical energy (40), wherein the at least one heating element (20) is connected to the supply of electrical energy (40).

**Patentansprüche**

1. Aerosolerzeugungssystem (100), umfassend:

wenigstens ein Heizelement (20), das zur Aufnahme eines aerosolbildenden Substrats (2) konfiguriert ist, und eine Steuerung (30), die zum Steuern der Freisetzung von flüchtigen Verbindungen aus dem Aerosolerzeugungssystem (100) konfiguriert ist, wobei das aerosolbildende Substrat (2) bei Erhitzung eine Vielzahl flüchtiger Verbindungen freisetzt und wobei jede der Vielzahl flüchtiger Verbindungen eine Mindestfreisetzungstemperatur hat, oberhalb derer die flüchtige Verbindung freigesetzt wird, wobei die Steuerung Folgendes aufweist:

Mittel zum Auswählen einer vorbestimmten maximalen Betriebstemperatur, wobei die vorbestimmte maximale Betriebstemperatur unter der Mindestfreisetzungstemperatur von wenigstens einer der flüchtigen Verbindungen ist, um ihre Freisetzung aus dem aerosolbildenden Substrat (2) zu verhindern, Mittel zum Regeln der Temperatur des wenigstens einen Heizelements (20), so dass wenigstens eine flüchtige Verbindung freigesetzt wird, und Mittel zum Messen des spezifischen Widerstands des wenigstens einen Heizelements (20), wobei die Steuerung (30) ferner konfiguriert ist zum:

Ableiten eines Ist-Betriebstemperaturwertes des wenigstens einen Heizelements (20) von dem Messwert des spezifischen Widerstands, Vergleichen des Ist-Betriebstemperaturwertes mit der vorbestimmten maximalen Betriebstemperatur und Einstellen der Temperatur des wenigstens einen Heizelements (20), um die Ist-Betriebstemperatur des wenigstens einen Heizelements (20) unter der vorbestimmten maximalen Betriebstemperatur zu halten.

2. System nach Anspruch 1, wobei das wenigstens eine Heizelement (20) ein Keramikmaterial aufweist.

3. System nach Anspruch 2, wobei das Heizelement (20) ferner ein Metall aufweist.

4. System nach Anspruch 3, wobei das Keramikmaterial und das Metall ein Verbundmaterial bilden, welches das wenigstens eine Heizelement (20) aufweist.

5. System nach Anspruch 3 oder 4, wobei das Metall das Keramikmaterial überzieht.

6. System nach einem der Ansprüche 3, 4 oder 5, wobei das Metall aus der Platingruppe ist.

7. System nach einem der Ansprüche 3 bis 6, wobei das wenigstens eine Heizelement (20) in der Form einer Heizlamelle bereitgestellt ist.

8. System nach einem der vorhergehenden Ansprüche, wobei das wenigstens eine Heizelement (20) elektrisch ist.

9. System nach Anspruch 8, das ferner eine elektrische Energieversorgung (40) aufweist, wobei das wenigstens eine Heizelement (20) mit der elektrischen Energieversorgung (40) verbunden ist.

**Revendications**

1. Système de génération d'aérosol (100) comprenant :

au moins un élément chauffant (20) configuré pour recevoir un substrat formant un aérosol (2); et un régulateur (30) configuré pour contrôler le dégagement de composés volatils du système de génération d'aérosol (100); dans lequel le substrat formant un aérosol (2) dégage une pluralité de composés volatils en chauffant et dans lequel chacun de la pluralité des composés volatils a une température minimale de dégagement au-dessus de laquelle le composé volatil se dégage, le régulateur (30) comprenant :

un moyen pour sélectionner une température maximale de fonctionnement prédéterminée, dans lequel la température maximale de fonctionnement prédéterminée est au-dessous de la température minimale de dégagement d'au moins l'un des composés volatils, afin d'empêcher son dégagement du substrat formant un aérosol (2);

un moyen pour contrôler la température de l'au moins un élément chauffant (20) de sorte qu'au moins un composé volatil se dégage; et

un moyen pour mesurer la résistivité de l'au moins un élément chauffant (20),

dans lequel le régulateur (30) est configuré en outre pour :

dériver une valeur de température réelle de fonctionnement de l'au moins un élément chauffant (20, de la mesure de résistivité;

comparer la valeur de la température réelle de fonctionnement à la température maximale de fonctionnement prédéterminée; et

ajuster la température de l'au moins un élément chauffant (20) afin de garder la température réelle de fonctionnement de l'au moins un élément chauffant (20) au-dessous de la température maximale de fonctionnement prédéterminée.

2. Système selon la revendication 1, dans lequel le au moins un élément chauffant (20) comprend une matière céramique.

3. Système selon la revendication 2, dans lequel l'élément chauffant (20) comprend en outre un métal.

4. Système selon la revendication 3, dans lequel la matière céramique et le métal forment un matériau composite qui comprend le au moins un élément chauffant (20).

5. Système selon la revendication 3 ou 4, dans lequel le métal recouvre la matière céramique.

6. Système selon l'une quelconque des revendications 3, 4 ou 5, dans lequel le métal est du groupe du platine.

7. Système selon l'une quelconque des revendications 3 à 6, dans lequel le au moins un élément chauffant (20) est fourni sous forme d'une lame chauffante.

8. Système selon l'une quelconque des revendications précédentes, dans lequel le au moins un élément chauffant (20) est électrique.

9. Système selon la revendication 8, comprenant en outre une alimentation en énergie électrique (40), dans lequel le au moins un élément chauffant (20) est connecté à l'alimentation en énergie électrique (40).

**Fig. 1**

**Fig. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03070031 A
- US 5060671 A
- US 5144962 A
- US 5372148 A
- US 5388594 A
- US 5498855 A
- US 5499636 A
- US 5505214 A
- US 5530225 A
- US 5591368 A
- US 5665262 A
- US 5666976 A
- US 5666978 A
- US 5692291 A
- US 5692525 A
- US 5708258 A
- US 5750964 A
- US 5902501 A
- US 5915387 A
- US 5934289 A
- US 5954979 A
- US 5967148 A

- US 5988176 A
- US 6026820 A
- US 6040560 A
- EP 1750788 A
- EP 1439876 A
- EP 0277519 A
- US 5396911 A
- EP 0857431 A
- EP 0893071 A
- WO 2007024130 A
- WO 2007066374 A
- EP 1736062 A
- WO 2007131449 A
- WO 2007131450 A
- WO 2007077167 A
- WO 03095688 A
- US 5514630 A
- KR 100636287 A
- JP 2006320286 A
- EP 1736065 A
- WO 2007078273 A